# EUROPEAN PATENT SPECIFICATION

(11) **EP 4 052 646 B1**
(45) Date of publication and mention of the grant of the patent: **09.04.2025**
(21) Application number: 22159118.3
(22) Date of filing: 28.02.2022
(51) Int. Cl.: A61B 5/0537, A61B 5/107

(54) **METHOD OF BIOIMPEDANCE VECTOR ANALYSIS TECHNOLOGY TO EVALUATE LOCAL OR WHOLE BODY BONE MINERAL DENSITY**
VERFAHREN ZUR BIOIMPEDANZ-VEKTORANALYSETECHNIK ZUR BEWERTUNG LOKALER ODER GANZKÖRPER-KNOCHENMINERALDICHTE
PROCÉDÉ DE TECHNOLOGIE D'ANALYSE DE VECTEUR DE BIOIMPÉDANCE POUR ÉVALUER LA DENSITÉ MINÉRALE OSSEUSE LOCALE OU DU CORPS ENTIER

(30) Priority: 05.03.2021 TW 110107861
(43) Date of publication of application: 07.09.2022
(73) Proprietor: Starbia Meditek Co., Ltd., Taichung City (TW)
(72) Inventor: Hsieh, Kuen-Chang, TAICHUNG CITY (TW)
(74) Representative: Straus, Alexander

(56) References cited:
- KR-A- 20200 023 915
- US-A1- 2002 156 378
- ANTUNES MELISSA ET AL: "Total and regional bone mineral density are associated with cellular health in older men and women", ARCHIVES OF GERONTOLOGY AND GERIATRICS, ELSEVIER, AMSTERDAM, NL, vol. 90, 15 June 2020 (2020-06-15), XP086246960, ISSN: 0167-4943, [retrieved on 20200615], DOI: 10.1016/J.ARCHGER.2020.104156

## Description

### BACKGROUND OF THE INVENTION

### 1. Field of the Invention

The present invention is related to bone mineral density evaluation technology, and particularly refers to a method of bioimpedance vector analysis technology to evaluate local or whole body bone mineral density.

### 2. Description of the Related Art

Most of the current bone mass inspection methods are measured according to the different absorption levels of ionized radiation by bone and soft tissue. Among them, the use of dual energy X-ray absorptiometry (DXA) to express bone mineral density (BMD) by mineral mass (g/cm²) is widely accepted.

However, although the method of applying DXA to measuring BMD is accepted by everyone, because DXA measurement is expensive, and its measurement location must be implemented in a hospital or related professional institution, it cannot be applied to home health care. In addition, each measurement of DXA takes tens of minutes. Therefore, the current method of measuring bone mineral density using DXA is costly and time-consuming, and it is not convenient to use. A method of bone mineral density evaluation according to the state of the art is for instance described in KR20200023915A.

### SUMMARY OF THE INVENTION

The present invention has been accomplished under the circumstances in view. It is the main object of the present invention to provide a method of bioimpedance vector analysis technology to evaluate local or whole body bone mineral density, which, compared with the prior art, is low in cost, fast, and convenient in use.

In order to achieve the above-mentioned purpose, a method of bioimpedance vector analysis technology to evaluate local or whole body bone mineral density provided by the present invention comprises the following steps:
Step (a) Measure the resistance (R) and reactance (Xc) of a subject with a bioimpedance measuring instrument, obtain the height of the subject, and then divide the height of the subject by the resistance to obtain a first value, and divide the height of the subject by the reactance to obtain a second value.
Step (b) Compare the first value and the second value with a bioimpedance vector analysis chart by the bioimpedance measuring instrument to evaluate the local or whole body bone mineral density of the subject.

Thereby, the method of bioimpedance vector analysis technology to evaluate local or whole body bone mineral density provided by the present invention only needs to obtain the height, resistance and reactance of the subject and then to obtain the first value and the second value, and then to compare the first value and the second value with a bioimpedance vector analysis chart, thereby evaluating the local or whole body bone mineral density of the subject. Compared with the prior art, the present invention is low in cost, fast, and convenient in use.

### BRIEF DESCRIPTION OF THE DRAWINGS

FIG. 1 is a flowchart of a preferred embodiment of the present invention.
FIG. 2 is a block diagram of a preferred embodiment of the present invention.
FIG. 3a is a bioimpedance vector analysis diagram of a preferred embodiment of the present invention, showing that the bone mineral densities of the whole bodies of the subjects are divided into six groups.
FIG. 3b is a bioimpedance vector analysis diagram of a preferred embodiment of the present invention, showing that the bone mineral densities of the lumbar spines of the subjects are divided into six groups.
FIG. 3c is a bioimpedance vector analysis diagram of a preferred embodiment of the present invention, showing that the bone mineral densities of the right upper limbs of the subjects are divided into six groups.
FIG. 4 is a GLM regression analysis diagram of a preferred embodiment of the present invention.

### DETAILED DESCRIPTION OF THE INVENTION

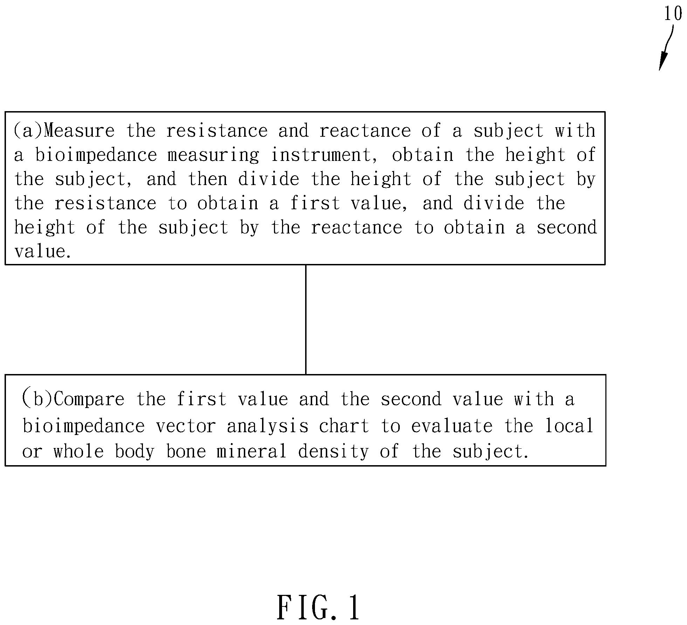

In order to explain the technical features of the present invention in detail, the following is a preferred embodiment, and the descriptions are as follows in conjunction with FIGS. 1-4. The method of bioimpedance vector analysis technology to evaluate local or whole body bone mineral density **10** provided by the present invention comprises the steps of (FIG. 1):
Step (a) Measure the resistance (R) and reactance (Xc) of a subject with a bioimpedance measuring instrument **20,** obtain the height of the subject, and then divide the height of the subject by the resistance to obtain a first value, and divide the height of the subject by the reactance to obtain a second value.
Step (b) Compare the first value and the second value with a bioimpedance vector analysis chart to evaluate the local or whole body bone mineral density of the subject.

In this preferred embodiment, the height of the subject is measured by a height rod and manually inputted into the bioimpedance measuring instrument **20,** and the accuracy of the height rod is set to 0.5 cm (but not limited to this). Thereby, the height that best meets the current situation of the subject can be obtained, so as to improve the accuracy of the evaluation result of the present invention. In other preferred embodiments, it can also be measured by other instruments that can measure the resistance and reactance of the subject. The subject's height can also be directly provided by the subject, and transmitted to the bioimpedance measuring instrument **20** in an automatic and wired/wireless manner. Therefore, the selection of the bioimpedance measuring instrument **20** and the way the bioimpedance measuring instrument **20** obtains the height of the subject are not limited to the present preferred embodiment.

In the present preferred embodiment, the bioimpedance measuring instrument **20** measures the body mineral density of the subject's body (BMDₜₒₜₐₗ), lumbar spine (BMD_{LS}) and right upper limb (BMD_{right arm}), a total of three parts of bone mineral density. In other preferred embodiments, it is also possible to choose to measure other parts of the subject according to needs, and to increase or decrease the measured parts of the subject according to needs.

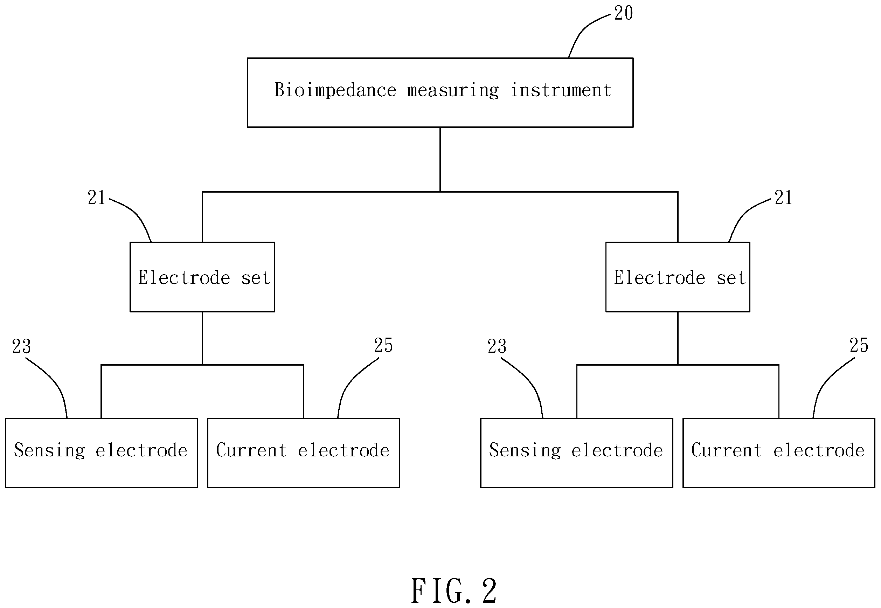

As shown in Figure 2, in the preferred embodiment, the bioimpedance measuring instrument **20** measures the resistance and reactance of the subject, and the subject performs the measurement of the bioimpedance measuring instrument **20** in a supine position, thereby reducing evaluation error. For the plural electrode sets **21** of the bioimpedance measuring instrument **20,** the number of the plural electrode sets **21** is two as an example. The plural electrode sets **21** contact the subject's right hand and right foot to form a measurement loop. Each electrode set **21** comprises a sensing electrode **23** and a current electrode **25.** The sensing electrode **23** and the current electrode **25** are separated by 5 cm, based on the characteristics of bioimpedance measurement, thereby improving the accuracy of the evaluation of the present invention. In other preferred embodiments, the subject can also perform the measurement of the bioimpedance measuring instrument **20** in a standing position. The plural electrode sets **21** can also be measured by contacting the subject's hands, feet, right hand to left foot, left hand to right foot, left hand to left foot, etc. Under different measurement modes, the number of the plural electrode sets **21** can also exceed two. The sensing electrode **23** and the current electrode **25** can also be between 1-5 cm or 5-10 cm. Therefore, the number of the plural electrode sets 21 and the manner in which the subject conducts the resistance and reactance measured by the bioimpedance measuring instrument **20** are not limited to this preferred embodiment.

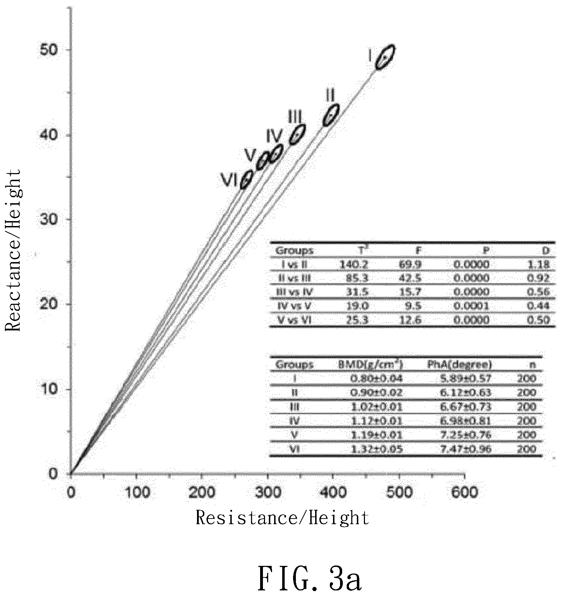

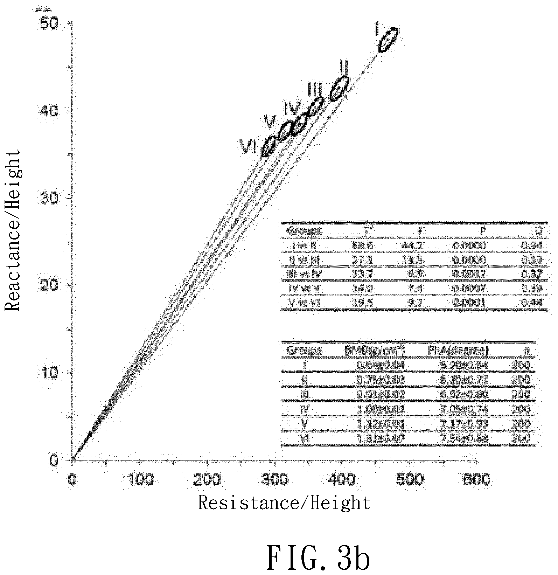

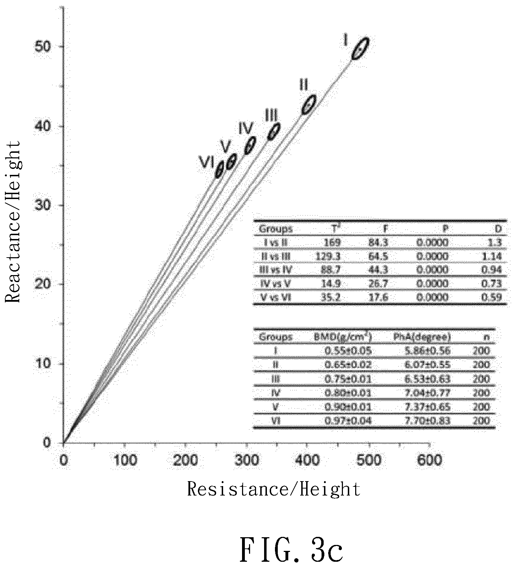

As shown in FIGS. 3a-c, in this preferred embodiment, the bioimpedance vector analysis diagram is to measure the electrical resistance and reactance of the whole body (FIG. 3a), lumbar spine (FIG. 3b), and upper right limb (FIG. 3c) of hundreds of subjects, and to calculate the impedance (R²+Xc²) ^{0.5} and get the bone mineral density of the whole body, lumbar spine, and right upper limb, and then to divide the three parts into six groups (I, II, III, IV, V, VI) according to the measured bone mineral density. Thereby, the subject only needs to obtain the height, resistance and reactance by the bioimpedance measuring instrument **20** to obtain the first value and the second value. After the first value and the second value are obtained, the whole body, lumbar spine, and right upper limb of the subject are corresponded to FIG. 3a, FIG. 3b, and FIG. 3c, respectively. It can then know which group the bone mineral density of the tested part of the subject is located in, and then evaluate the bone mineral density of the subject. In other preferred embodiments, it is also possible to increase or decrease the measuring parts of the subject according to needs, and divide the size of bone mineral density into more or less groups according to needs. Therefore, the use of the bioimpedance vector analysis diagram is not limited to this preferred embodiment.

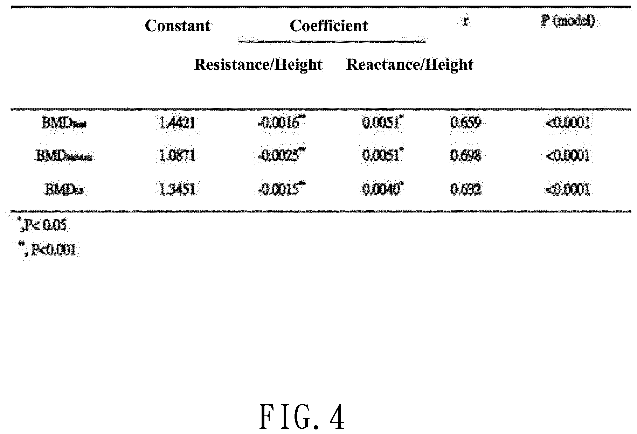

In order to verify the accuracy of evaluating bone mineral density with the method of the present invention. With dual energy X-ray absorptiometry (DXA), the whole body, lumbar spine and right upper limb of the aforementioned subjects were measured, and the average bone mineral density was taken. The average bone mineral density thus obtained and the average value of bone mineral density measured by the present invention, as shown in FIG. 4, in the GLM regression analysis, the corresponding weighting coefficients and correlations show that the R/H and Xc/H values of the subject are positively correlated with the values of the whole body, lumbar spine and right upper limb measured by DXA.

In other preferred embodiments, to verify the accuracy of the present invention, the bio-impedance vector analysis diagram can also be obtained after measurement by a quantitative ultrasonic inspection instrument, quantitative computed tomography, or traditional X-ray inspection. Therefore, the acquisition of the bioimpedance vector analysis diagram is not limited to this preferred embodiment.

Thereby, the method of bioimpedance vector analysis technology to evaluate local or whole body bone mineral density provided by the present invention only needs to obtain the height, resistance and reactance of the object so as to obtain a first value and a second value, and after obtaining the first value and the second value, it only needs compare the bioimpedance vector analysis diagram to evaluate the bone mineral density of the subject. Compared with the prior art, the present invention is low in cost, fast, and convenient in use.

In addition, the method of bioimpedance vector analysis technology to evaluate local or whole body bone mineral density provided by the present invention, when obtaining the height, resistance and reactance of the subject at different time points, and obtaining the first value and the second value at each time point, and then comparing the bioimpedance vector analysis diagram, it is also convenient to be able to evaluate the changes in the bone mineral density of the subject.

## Claims

1. A method of bioimpedance vector analysis technology to evaluate local or whole body bone mineral density (10), comprising the steps of:
(a) measuring a resistance and a reactance of a subject with a bioimpedance measuring instrument (20), obtaining a height of said subject, and then dividing the height of said subject by said resistance to obtain a first value, and dividing the height of said subject by said reactance to obtain a second value; and
(b) comparing said first value and said second value with a bioimpedance vector analysis chart by the bioimpedance measuring instrument (20) to evaluate the local or whole body bone mineral density of said subject.

2. The method of bioimpedance vector analysis technology to evaluate local or whole body bone mineral density (10) as claimed in claim 1, wherein in step (a), the method for said bioimpedance measuring instrument to measure the resistance and reactance of said subject is to measure plural electrode sets (21) of said bioimpedance measuring instrument, said plural electrode sets (21) contacting the hands and feet, hands or feet of said subject to form a measurement loop, each said electrode set comprising a sensing electrode (23) and a current electrode (25), said sensing electrode (23) and said current electrode (25) being 1-10 cm apart.

3. The method of bioimpedance vector analysis technology to evaluate local or whole body bone mineral density (10) as claimed in claim 1, wherein in step (a), said subject is to perform the measurement of said bioimpedance measuring instrument in a supine, standing or sitting position selectively.

## Patentansprüche

1. Verfahren der Bioimpedanz-Vektoranalysetechnik zur Bewertung der lokalen oder Ganzkörper-Knochenmineraldichte (10), welches die Schritte umfasst:
(a) Erfassen eines Widerstands und einer Reaktanz einer Person mit einem Bioimpedanz-Messinstrument (20), Ermitteln einer Höhe der Person und anschließendes Dividieren der Höhe der Person durch den Widerstand, um einen ersten Wert zu erhalten, und Dividieren der Höhe der Person durch die Reaktanz, um einen zweiten Wert zu erhalten; und
(b) Vergleichen des ersten Wertes und des zweiten Wertes mit einem Bioimpedanz-Vektoranalysechart durch das Bioimpedanz-Messinstrument (20), um die lokale oder Ganzkörper-Knochenmineraldichte der Person zu bewerten.

2. Verfahren der Bioimpedanz-Vektoranalysetechnik zur Bewertung der lokalen oder Ganzkörper-Knochenmineraldichte (10) nach Anspruch 1, wobei in Schritt (a) das Verfahren für das Bioimpedanz-Messinstrument zur Messung des Widerstands und der Reaktanz der Person darin besteht, mehrere Elektrodensätze (21) des Bioimpedanz-Messinstruments zu messen, worin die mehreren Elektrodensätze (21) die Hände und Füße, Hände oder Füße der Person berühren, um eine Messschleife zu bilden, worin jeder Elektrodensatz eine Sensorelektrode (23) und eine Stromelektrode (25) umfasst, wobei die Sensorelektrode (23) und die Stromelektrode (25) 1-10 cm voneinander entfernt sind.

3. Verfahren der Bioimpedanz-Vektoranalysetechnik zur Bewertung der lokalen oder Ganzkörper-Knochenmineraldichte (10) nach Anspruch 1, wobei in Schritt (a) die Erfassung des Bioimpedanz-Messinstruments an der Person wahlweise in einer Rücken-, Steh- oder Sitzposition durchführt wird.

## Revendications

1. Méthode d'analyse vectorielle de la bioimpédance pour évaluer la densité minérale osseuse locale ou du corps entier (10), comprenant les étapes suivantes:
(a) mesurer une résistance et une réactance d'un sujet à l'aide d'un instrument de mesure de la bioimpédance (20), obtenir la hauteur dudit sujet, puis diviser la hauteur dudit sujet par ladite résistance pour obtenir une première valeur, et diviser la hauteur dudit sujet par ladite réactance pour obtenir une seconde valeur; et
(b) comparer ladite première valeur et ladite seconde valeur à un graphique d'analyse vectorielle de la bioimpédance établi par l'instrument de mesure de la bioimpédance (20) afin d'évaluer la densité minérale osseuse locale ou du corps entier dudit sujet.

2. Méthode d'analyse vectorielle de la bioimpédance pour évaluer la densité minérale osseuse locale ou du corps entier (10) selon la revendication 1, dans laquelle à l'étape (a), la méthode pour ledit instrument de mesure de la bioimpédance de mesurer la résistance et la réactance dudit sujet consiste à mesurer plusieurs ensembles d'électrodes (21) dudit instrument de mesure de la bioimpédance, ces ensembles d'électrodes (21) sont en contact avec les mains et les pieds, les mains ou les pieds du sujet pour former une boucle de mesure, chaque ensemble d'électrodes comprenant une électrode de détection (23) et une électrode de courant (25), l'électrode de détection (23) et l'électrode de courant (25) étant distantes de 1-10 cm l'une de l'autre.

3. Méthode d'analyse vectorielle de la bioimpédance pour évaluer la densité minérale osseuse locale ou du corps entier (10) selon la revendication 1, dans laquelle, à l'étape (a), le sujet doit effectuer la mesure de l'instrument de mesure de la bioimpédance en position couchée, debout ou assise, au choix.
